# EUROPEAN PATENT APPLICATION

(11) **EP 3 305 167 A1**
(43) Date of publication of application: **11.04.2018**
(21) Application number: 16802852.0
(22) Date of filing: 23.02.2016
(51) Int. Cl.: A61B 1/00, G02B 23/24

(54) **MOUNTING UNIT**

(30) Priority: 03.06.2015 JP 2015113342
(71) Applicant: Olympus Corporation, Shibuya-ku Tokyo 151-0072 (JP)
(72) Inventor: OKAMOTO Yasuhiro, Tokyo 192-8507 (JP)
(74) Representative: Schicker, Silvia
(86) International application number: PCT/JP2016/055180
(87) International publication number: WO 2016/194413

(57) **Abstract**

An attachment unit 30 includes a tube main body 31 attached to an insertion section 3 of an endoscope 2 and disposed to be rotatable around a longitudinal axis 3Aa of the insertion section 3 and a fin section 32 protrudingly provided on an outer circumferential surface of the tube main body 31 and spirally extended along a longitudinal axis of the tube main body. In the fin section 32, a first force amount F1 necessary for bringing down the fin section 32 toward a distal end side Yf direction and a second force amount F2 necessary for bringing down the fin section 32 toward a proximal end side Yr direction, which is an opposite direction of the distal end side, are different. In the fin section 32 provided on the tube main body 31, the first force amount f1 is larger than the second force amount F2.

## Description

### Technical Field

The present invention relates to an attachment unit attached to an insertion section of an insertion device, which is inserted into a tested part, and capable of rotating around an axis of the insertion section.

### Background Art

Endoscopes are used in a medical field, an industrial field, and the like.

The endoscope for medical use can perform observation of organs and the like by inserting an insertion section into a body, which is a tested part.

A general endoscope includes an operation section and an insertion section. The insertion section of the endoscope is inserted into a digestive organ digestive tract per anum, per os, or per nasal. The insertion section is extended from the operation section. A flexible tube section having flexibility, a bending section bendable in a left-right direction and an up-down direction, and a distal end section are concatenated in order from the operation section side.

Operation buttons, a bending operation knob, and the like, which a surgeon can operate with gripping fingers, are provided in the operation section. The bending section of the insertion section is bent in the up-down and left-right directions according to operation of the bending operation knob provided in the operation section.

When the surgeon inserts the insertion section into, for example, an intestinal tract, the surgeon performs twisting operation or feeding operation of the insertion section located outside a body and advances the insertion section toward an intestinal tract depth while operating the bending operation knob provided in the operation section to bend the bending section.

However, the intestinal tract is soft and long and complexly curves. The intestinal tract is not firmly fixed in the body. Therefore, even if the surgeon advances the insertion section while compressing the intestinal tract making full use of the twisting operation, the feeding operation, the operation for bending the bending section, and the like, the insertion section is sometimes returned to an original position of the insertion section by a reaction from the compressed intestinal tract that is returning to an original state of the intestinal tract.

In particular, the reaction from the intestinal tract is larger as the insertion section is inserted deeper in the intestinal tract. The surgeon needs to acquire skill to be able to cause the insertion section to reach a target intestinal tract depth while retaining a compressed state.

Japanese Patent No. 5326049 discloses an attachment unit attached to an insertion section in a state in which the attachment unit is rotatable around a longitudinal axis. The attachment unit includes a tube main body extended along the longitudinal axis and a fin section spirally extended along the longitudinal axis in an outer circumferential section of a tube main body.

The fin section of the attachment unit rotatably attached to the insertion section comes into contact with a lumen wall when the insertion section is inserted into a lumen such as a large intestine. In a state of the contact, when the tube main body of the attachment unit is rotated, for example, clockwise around the longitudinal axis of the insertion section when viewed from a proximal end side of the insertion section, propulsion for advancing the insertion section to a distal end side is given from the attachment unit to the insertion section. On the other hand, when the tube main body is rotated counterclockwise, propulsion for retracting the insertion section to a proximal end side is given from the attachment unit to the insertion section.

With the endoscope in which the attachment unit is attached to the insertion section, when the surgeon performs hand-side operation for advancing the insertion section, the surgeon is capable of smoothly causing the insertion section to reach the intestinal tract depth by obtaining the propulsion for advancing the insertion section to the distal end side.

In the attachment unit, the fin section includes a first width dimension section and a second width dimension section. A width dimension of the second width dimension section is set smaller than a width dimension of the first width dimension section. The second width dimension section bends when an external force acts on the fin section in a direction parallel to the longitudinal axis.

Therefore, an outer diameter dimension to an outer circumferential end of the fin section changes to a diameter smaller than an original dimension in a bent state.

However, when the insertion section is advanced toward the intestinal tract depth, reaction from the intestinal tract compressed by passing the fin section of the attachment unit disclosed in Japanese Patent No. 5326049 always acts on the fin section. Therefore, the second width dimension section is likely to be bent in the opposite direction of the direction described above by the reaction applied to the fin section from the compressed intestinal tract.

When the second width dimension section is bent in the opposite direction by the reaction from the compressed intestinal tract, the compressed intestinal tract climbs over the fin section and the insertion section is returned to an original position.

The present invention has been devised in view of the above circumstances, and an object of the invention is to provide an attachment unit that, while compressing an intestinal tract having flexibility by giving, to an insertion section, propulsion for advancing the insertion section to an intestinal tract depth, surely retains a compressed state of the compressed intestinal tract and advances the insertion section.

### Disclosure of Invention

### Means for Solving the Problem

An attachment unit in an aspect of the present invention includes: a unit main body provided in an insertion section of an insertion device and disposed to be rotatable around a longitudinal axis of the insertion section; and an elastic convex section protrudingly provided on an outer circumferential surface of the unit main body and spirally extended along a longitudinal axis of the unit main body. In the elastic convex section, a first force amount necessary for bringing down the elastic convex section to a distal end side of the insertion section and a second force amount necessary for bringing down the elastic convex section to a proximal end side, which is an opposite direction of the distal end side, are different. In the elastic convex section provided in the unit main body, the first force amount is larger than the second force amount.

### Advantageous Effects of Invention

According to the present invention, it is possible to realize an attachment unit that, while compressing an intestinal tract having flexibility by giving, to an insertion section, propulsion for advancing the insertion section to an intestinal tract depth, surely retains a compressed state of the compressed intestinal tract and advances the insertion section.

### Brief Description of the Drawings

Fig. 1 is a diagram for explaining an endoscope system including an endoscope and an attachment unit;
Fig. 2 is a diagram for explaining the endoscope in which the attachment unit is attached to an insertion section;
Fig. 3 is a sectional view indicated by a Y3-Y3 line in Fig. 2 and is a diagram for explaining a relation between a unit main body and a fin section configuring the attachment unit;
Fig. 4 is a diagram for explaining an electric driving source that rotates the attachment unit and the unit main body of the attachment unit;
Fig. 5 is a Y4-Y4 line sectional view of Fig. 4;
Fig. 6A is a diagram showing a state in which the insertion section provided with the attachment unit is inserted into a large intestine;
Fig. 6B is a diagram showing a state in which the rotating attachment unit advances the insertion section while drawing in a lumen wall;
Fig. 6C is a diagram showing a state in which the lumen wall is further drawn in and the insertion section is further advanced toward a depth;
Fig. 6D is a diagram showing a state in which a distal end portion of the insertion section provided with the attachment unit reaches a depth of the large intestine;
Fig. 7A is an explanatory diagram for explaining another configuration example in which the fin section less easily falls toward an insertion section distal end side direction and is an explanatory diagram showing a configuration example in which the fin section is fixed to an outer surface of a tube main body using a first adhesive and a second adhesive;
Fig. 7B is an explanatory diagram for explaining another configuration example in which the fin section less easily falls toward the insertion section distal end side direction and is an explanatory diagram showing a configuration example in which the fin section is fixed to the outer surface of the tube main body using the first adhesive;
Fig. 8A is an explanatory diagram for explaining another configuration example in which the fin section less easily falls toward the insertion section distal end side direction and is an explanatory diagram showing an example in which an adhesive is applied along a longitudinal direction on a first side surface side and a second side surface side of a fin member;
Fig. 8B is an explanatory diagram for explaining another configuration example in which the fin section less easily falls toward the insertion section distal end side direction and is an explanatory diagram showing an example in which the adhesive is applied to a fixed surface to fix the fin member to the tube main body with the adhesive;
Fig. 8C is an explanatory diagram for explaining another configuration example in which the fin section less easily falls toward the insertion section distal end side direction and is an explanatory diagram showing an example in which the adhesive is applied to only an insertion section distal end side of the fixed surface to bond and fix the fin member to the outer surface of the tube main body;
Fig. 9A is an explanatory diagram for explaining another configuration example of the fin member and is an explanatory diagram showing a configuration example in which the fin member includes, along a longitudinal axis, a hollow section having a sectional shape decided in advance;
Fig. 9B is an explanatory diagram for explaining another configuration example of the fin member and is an explanatory diagram showing an example in which two elastic members are integrally configured;
Fig. 9C is an explanatory diagram for explaining another configuration example of the fin member and is an explanatory diagram showing an example in which a hollow section is provided in the fin member;
Fig. 10A is an explanatory diagram for explaining an example of fixing of the fin member to the unit main body and is an explanatory diagram showing an example in which the fin member is bonded and fixed by an adhesive applied to a first side surface and an adhesive applied to a second side surface;
Fig. 10B is an explanatory diagram showing an example in which a first elastic member is formed thick and a second elastic member is formed thin;
Fig. 10C is an explanatory diagram showing an example in which a hollow section is provided in the fin member; and
Fig. 10D is an explanatory diagram showing an example in which bonding and fixing sections are provided on the first side surface side and the second side surface side of the fin member.

### Best Mode for Carrying Out the Invention

An embodiment of the present invention is explained below with reference to the drawings.

Note that, in the figures referred to below in the explanation, scales are sometimes differentiated for each of components to show the components in recognizable sizes on the drawings. That is, the present invention is not limited to only numbers of the components, shapes of the components, ratios of the sizes of the components, and relative positional relations among the components described in the figured.

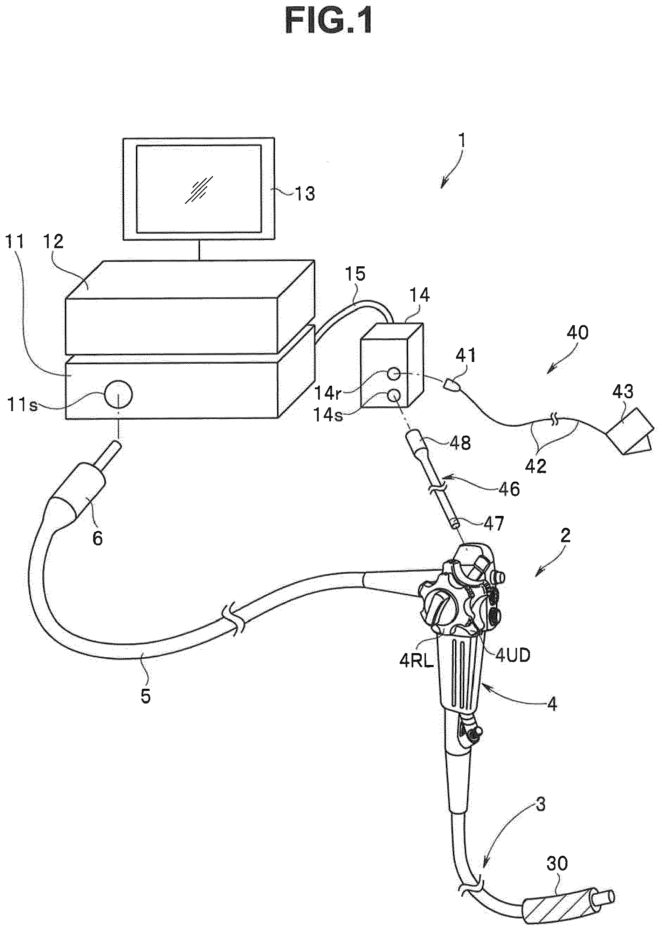

In the present embodiment, an insertion device is an endoscope 2 shown in Fig. 1. Therefore, a main part of an endoscope system 1 is configured to include the endoscope 2 and a light source device 11, a processor for display 12, a monitor 13, and a control device 14, which are endoscope external devices.

The endoscope 2 includes an insertion section 3 explained below. An attachment unit for an endoscope (hereinafter abbreviated as attachment unit) 30 explained below is provided in the insertion section 3.

Reference numeral 15 denotes a connection cable, which electrically connects the light source device 11 and the control device 14. The control device 14 incorporates a control section (not shown in the figure) for, for example, electrically controlling the attachment unit 30 to be driven.

Reference numeral 40 denotes an external switch, which includes a foot-switch connecting section 41, a foot switch cable 42, and a foot switch section 43. The foot-switch connecting section 41 is configured to be detachably attachable to a foot-switch connection port 14r of the control device 14.

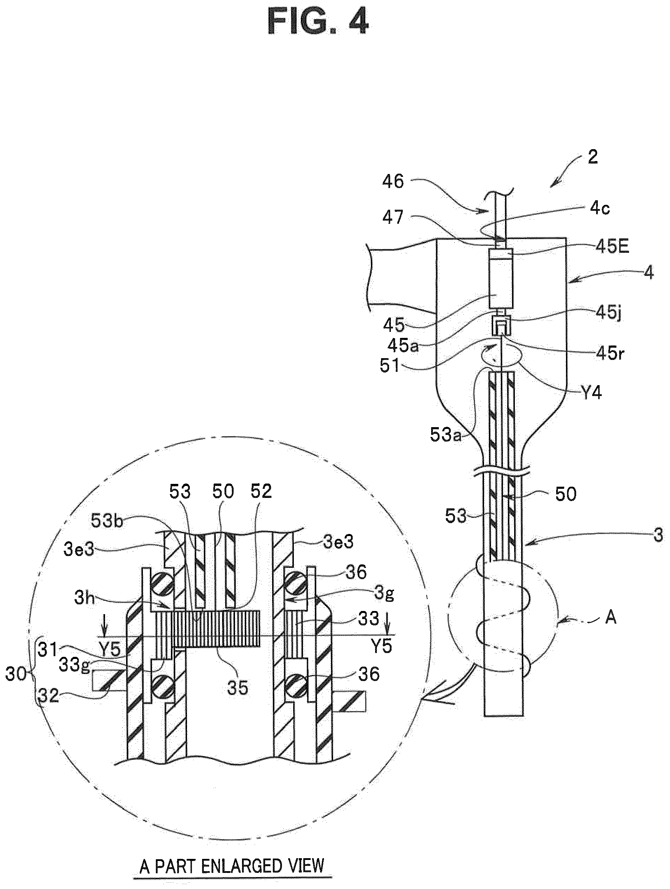

Reference numeral 46 denotes an electric cable, which includes a first connecting section 47 and a second connecting section 48. The first connecting section 47 is detachably attachable to an electric connecting section (reference sign 4c in Fig. 4) provided in an operation section 4 explained below. The second connecting section 48 is detachably attachable to a cable connection port 14s of the control device 14.

The light source device 11 is electrically connected to the processor for display 12 by a not-shown connection cable. The processor for display 12 is electrically connected to the monitor 13.

The endoscope 2 includes the elongated insertion section 3 inserted into digestive organ digestive tracts such as an esophagus, a stomach, a duodenum, a small intestine, and a large intestine. The operation section 4 is provided at a proximal end of the insertion section 3. A universal cord 5 extends from the operation section 4.

A connection connector 6 is provided at an extension end of the universal cord 5. The connection connector 6 is configured to be detachably attachable to a connector connecting section 11s of the light source device 11.

In the present embodiment, the endoscope 2 is, for example, an endoscope for a lower digestive tract. However, the endoscope 2 is not limited to the endoscope for a lower digestive tract and may be, for example, an endoscope for upper digestive tracts.

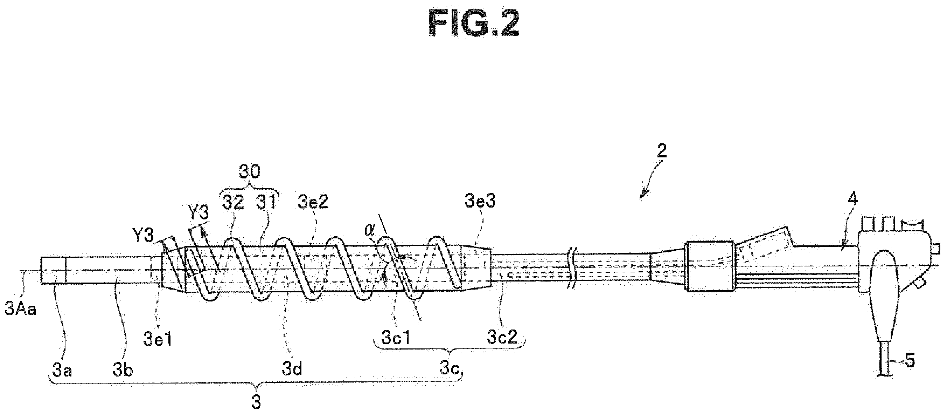

As shown in Fig. 2, the insertion section 3 includes a distal end portion 3a on a distal end side. A bending section 3b is provided on a proximal end side of the distal end portion 3a. A flexible tube section 3c having flexibility decided in advance is provided on the proximal end side of the bending section 3b. The bending section 3b is configured to be bendable, for example, in up-down and left-right directions.

Reference sign 3d denotes a passive bending section.

As shown in Fig. 1, an up-down bending operation knob 4UD and a left-right bending operation knob 4RL, which are bending operation devices, are provided in the operation section 4. The respective operation knobs 4UD and 4RL are configured to be respectively turnable around not-shown axes.

Like a bending section of an endoscope in the past, a bending wire (not shown in the figure) is towed according to operation of the up-down bending operation knob 4UD or the left-right bending operation knob 4RL, whereby the bending section 3b bends in a desired direction.

As shown in Fig. 1 and Fig. 2, the attachment unit 30 is provided in a distal end side outer circumference of the flexible tube section 3c configuring the insertion section 3. The attachment unit 30 is a driven section and is disposed to be turnable around a longitudinal axis of the insertion section 3 to perform a first motion and a second motion.

The first motion is a rotating motion for generating first propulsion for advancing the insertion section 3 toward the distal end side, that is, toward an intestinal tract depth. On the other hand, the second motion is a rotating motion for generating second propulsion for retracting the insertion section 3 toward the proximal end side, that is, from an inside of a body to an outside.

In the present embodiment, the attachment unit 30 is configured to rotate clockwise or counterclockwise according to operation of an external switch 40, when viewed from the proximal end side of the insertion section 3 around a longitudinal axis 3Aa of the insertion section 3 shown in Fig. 2.

The passive bending section 3d is configured to passively bend by receiving an external force. In the present embodiment, the passive bending section 3d is disposed between the bending section 3b and the flexible tube section 3c configuring the insertion section 3.

In the present embodiment, the flexible tube section 3c is configured of a first flexible tube 3c1 and a second flexible tube 3c2. The first flexible tube 3c1 is located on the passive bending section 3d side. The second flexible tube 3c2 is connected to a proximal end of the first flexible tube 3c 1.

The bending section 3b and the passive bending section 3d are connected via a first connection tube 3e1.

The passive bending section 3d and the first flexible tube 3c1 are connected via a second connection tube 3e2. The first flexible tube 3c1 and the second flexible tube 3c2 are connected via a third connection tube 3e3.

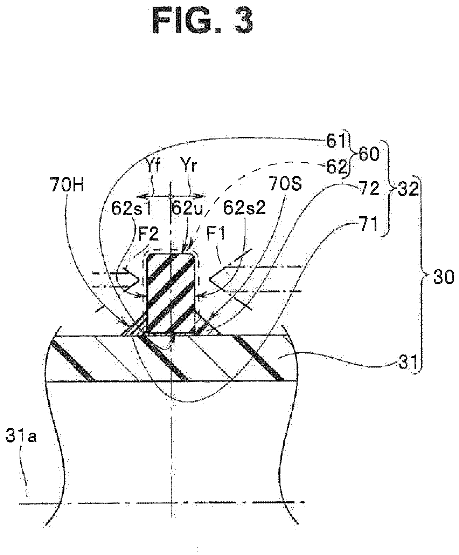

As shown in Fig. 2 and Fig. 3, the attachment unit 30 includes a tube main body 31, which is a unit main body, and a fin section 32, which is an elastic convex section.

The fin section 32 projects from an outer surface of the tube main body 31 toward a radial direction outer side of the tube main body 31 by an amount decided in advance. The fin section 32 is provided to spirally extend on the outer surface of the tube main body 31. An angle α of the spiral fin section 32 with respect to an axis 3Aa is set to, for example, an angle larger than 45°.

The tube main body 31 is a tube made of resin such as polyurethane and has flexibility and elasticity decided in advance. The tube main body 31 has an inner diameter for disposing the tube main body 31 in a state in which the tube main body 31 loosely fits in the outer circumferential surface of the insertion section 3.

A distal end portion of the tube main body 31 is disposed in a not-shown attachment groove of the first connection tube 3e1 also functioning as an attaching section of an insertion supporting mechanism section. On the other hand, as explained below, a proximal end section of the tube main body 31 is disposed in an attachment groove (see reference sign 3g in Fig. 4) of the third connection tube 3e3 also functioning as the attaching section of the insertion supporting mechanism section.

With this configuration, the tube main body 31 is rotatable clockwise and counterclockwise with respect to the insertion section 3.

The attachment unit 30 is configured to give propulsion generated by screw action due to contact of the spiral fin section 32 with a lumen wall to the insertion section 3 when the tube main body 31 is rotated in a winding direction around an axis or in an opposite direction of the winding direction with respect to the insertion section 3.

More specifically, in a state in which the fin section 32 is in contact with the lumen wall, when the tube main body 31 is rotated around the axis, that is, clockwise (right-handed), which is the same as the winding direction of the fin section 32, when viewed from the operation section 4 side, the attachment unit 30 of the present embodiment gives first propulsion for advancing the insertion section 3 toward a body cavity depth to the insertion section 3.

Conversely, in the state in which the fin section 32 is in contact with the lumen wall, when the tube main body 31 is rotated counterclockwise (left-handed), which is an opposite direction of the winding direction of the fin section 32 when viewed from the operation section 4 side, the attachment unit 30 gives second propulsion for retracting the insertion section 3 toward the outside of the body to the insertion section 3.

As shown in Fig. 3, in the present embodiment, the fin section 32 is formed by fixing a fin member 60 on the outer surface of the tube main body 31. That is, the fin member 60 is a member different from the tube main body 31.

The fin member 60 is an elongated and solid elastic bar-like member made of, for example, rubber having flexibility and elasticity decided in advance. A sectional shape of the fin member 60 is formed in a shape decided in advance.

The fin member 60 includes a fixed surface 61 and a contact surface 62. The fixed surface 61 is a surface disposed on the outer circumferential surface of the tube main body 31. On the other hand, the contact surface 62 is an outer side surface excluding the fixed surface 61 and is a surface in contact with the lumen wall. A ridge line where an upper surface 62u of the contact surface 62 and side surfaces 62s1 and 62s2 disposed across the upper surface 62u cross is chamfered and rounded.

As shown in Fig. 2 and Fig. 3, the fixed surface 61 of the fin member 60 is disposed in an erected state on the outer surface of the tube main body 31. In this disposition state, the fin member 60 is spirally extended along a longitudinal axis 31a of the tube main body 31.

In the present embodiment, the fin member 60 is bonded and fixed to the outer surface of the tube main body 31 by a first adhesive 71 and a second adhesive 72. The first adhesive 71 is applied to the first side surface 62s1, which is the proximal end side (an arrow Yr direction side in Fig. 3), along a longitudinal axis. On the other hand, the second adhesive 72 is applied to the second side surface 62s2, which is the distal end side (an arrow Yf direction side in Fig. 3), along a longitudinal direction. The fin member 60 is bonded and fixed to the outer surface of the tube main body 31, whereby the fin section 32 is provided in the tube main body 31.

The fin section 32 is a spiral wound clockwise (right-handed) from the operation section 4 side toward the distal end side when viewed from the proximal end side.

In the present embodiment, the first adhesive 71 and the second adhesive 72 are different adhesives. Hardness of an adhesive hardened section formed by hardening of an adhesive is different in the first adhesive 71 and the second adhesive 72.

More specifically, hardness of a first hardened section, in which the first adhesive 71 hardens, is set higher than hardness of a second hardened section formed by hardening of the second adhesive 72.

In this way, the fin member 60 is bonded and fixed to the tube main body 31 by the first adhesive 71 and the second adhesive 72. Consequently, a hard fixed section 70H, which is the first hardened section formed by the hardening of the first adhesive 71, is provided on the distal end side of the fin section 32. A soft fixed section 70S, which is the second hardened section formed by the hardening of the second adhesive 72, is provided on the proximal end side of the fin section 32.

Note that a sectional area of the hard fixed section 70H and a sectional area of the soft fixed section 70S have the same shape.

The hard fixed section 70H is provided on the distal end side of the fin section 32 fixed to the tube main body 31 by bonding in this way, and consequently, the fin section 32 less easily falls to the distal end side.

On the other hand, the soft fixed section 70S is provided on the proximal end side along the longitudinal axis 31a of the fin section 32, and consequently, the fin section 32 easily falls to the proximal end side.

Therefore, a first force amount F1 necessary for bringing down the fin section 32 to the distal end side and a second force amount F2 necessary for bringing down the fin section 32 to the proximal end side are different force amounts.

To bring down the fin section 32 to the distal end side, a force amount larger than a force amount for bringing down the fin section 32 to the proximal end side is necessary. That is, the first force amount F1 is a force amount larger than the second force amount F2.

In the present embodiment, when the insertion section 3 is advanced toward an intestinal tract depth while an intestinal tract is compressed, the first force amount F1 is a force amount larger in advance than reaction from the compressed intestinal tract returning to an original state.

On the other hand, the second force amount F2 is a force amount with which the fin section 32 is bent by an external force given to the fin section 32 from an intestinal tract wall when the insertion section 3 is advanced toward the intestinal tract depth.

With this configuration, the fin section 32 erected on the tube main body 31 is prevented from being brought down to the distal end side by the reaction from the compressed intestinal tract wall when the insertion section 3 advances to the intestinal tract depth.

Note that the tube main body 31 of the attachment unit 30 is rotated clockwise or counterclockwise by, for example, a driving motor 45, which is an electric driving source, disposed in the operation section 4 as shown in Fig. 4.

The driving motor 45 generates a rotation driving force for rotating the attachment unit 30. A driving shaft 45a of the driving motor 45 is rotatable clockwise or counterclockwise around an axis when viewed from a motor proximal end side as indicated by an arrow Y4.

A clockwise rotation driving force or a counterclockwise rotation driving force is transmitted to the attachment unit 30 by a driving shaft 50, which is a rotation driving force transmitting member.

The driving shaft 50 is inserted through and disposed in the flexible tube section 3c of the endoscope 2 along a longitudinal axis in a state in which the driving shaft 50 is covered by a soft protection tube 53. A first end portion 51 of the driving shaft 50 projects further than a first side end 53a of the protection tube 53. A second end portion 52 projects further than a second side end 53b of the protection tube 53.

The driving shaft 50 is a flexible shaft having flexibility decided in advance and is formed by winding a special hard steel wire or a stainless steel wire for spring alternately in right winding and left winding in several layers.

The first end portion 51, which is an end portion disposed in the operation section 4, of the driving shaft 50 is coupled to the driving shaft 45a of the motor 45. More specifically, a coupling section 45j is integrally fixed in the driving shaft 45a of the motor 45. The first end portion 51 of the driving shaft 50 is integrally fixed to a coupling rod 45r. The coupling rod 45r is engaged and arranged in the coupling section 45j to be capable of advancing and retracting in a longitudinal axis direction and capable of transmitting torque to the coupling section 45j.

The attachment unit 30 is configured to rotate clockwise or counterclockwise when viewed from the proximal end side of the operation section 4 according to switch operation of the external switch 40.

Note that the motor 45 is in a stopped state, for example, when the foot switch section 43 is in a non-step-in state. Rotating speed of the motor 45 may change according to magnitude of a step-in amount of the foot switch section 43.

With the endoscope system 1 explained above, an instruction signal is outputted to the control section of the control device 14 when the surgeon steps in the foot switch section 43. The control section generates a motor driving signal. The generated motor driving signal is outputted from the control device 14 to the motor 45 via the electric cable 46. As a result, the driving shaft 45a of the motor 45 is rotated clockwise or counterclockwise around the axis.

Then, the driving shaft 50 starts rotation according to the clockwise or counterclockwise rotation of the driving shaft 45a of the motor 45. That is, the driving shaft 45a of the motor 45 is driven to rotate clockwise, whereby the driving shaft 50 rotates in the same direction. The driving shaft 45a is driven to rotate counterclockwise, whereby the driving shaft 50 rotates in the same direction.

Note that a signal line connected to an encoder for motor 45E is inserted through the electric cable 46. The encoder for motor 45E detects a rotating direction and rotating speed of the motor 45 and outputs a detection signal to the control section of the control device 14 via the signal line in the electric cable 46.

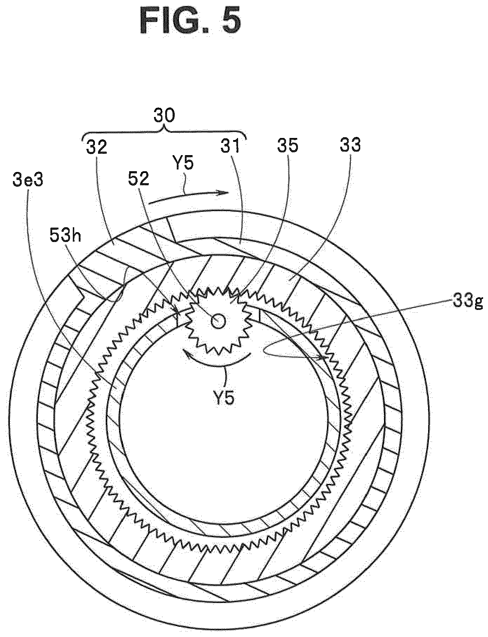

On the other hand, the second end portion 52, which is an end portion disposed in the flexible tube section 3c, of the driving shaft 50 is integrally fixed to a transmission gear 35. As shown in Fig. 4 and Fig. 5, the transmission gear 35 meshes with a gear section 33g. The gear section 33g is formed on an inner circumferential surface of an annular tube-main-body rotating section 33.

With this configuration, the transmission gear 53 is rotated in the same direction according to the rotation of the driving shaft 50. The tube-main-body rotating section 33 is rotated in the same direction according to the rotation of the transmission gear 35.

Note that the driving shaft 50 is not limited to the flexible shaft and may be a torque coil, which is a multi-line multi-layer coil, a torque wire, or the like as long as torsional rigidity at the time when the driving shaft 50 is rotated in the winding direction and torsional rigidity at the time when the driving shaft 50 is rotated in the opposite direction of the winding direction are different.

An outer circumferential surface of the tube-main-body rotating section 33 is integrally fixed to the tube main body 31 of the attachment unit 30. The gear section 33g is disposed to pass through a through-hole 3h, which causes an inside and an outside to communicate, provided in the third connection tube 3e3 and project outward from an outer circumferential surface of the third connection tube 3e3.

The width of the through-hole 3h is set to a dimension decided in advance taking into account a thickness dimension of the transmission gear 35 in order to restrict movement in the axis direction of the transmission gear 35.

Reference numeral 36 denotes an O-shaped ring. A pair of O-shaped rings 36 is disposed in close contact with an inner circumferential surface of the tube-main-body rotating section 33 and disposed in close contact with an outer circumferential surface of the third connection tube 3e3.

The tube-main-body rotating section 33 is integral with the tube main body 31 of the attachment unit 30 capable of turning with respect to the insertion section 3.

With this configuration, water tightness between the inner circumferential surface of the tube-main-body rotating section 33 and the outer circumferential surface of the third connection tube 3e3 is maintained.

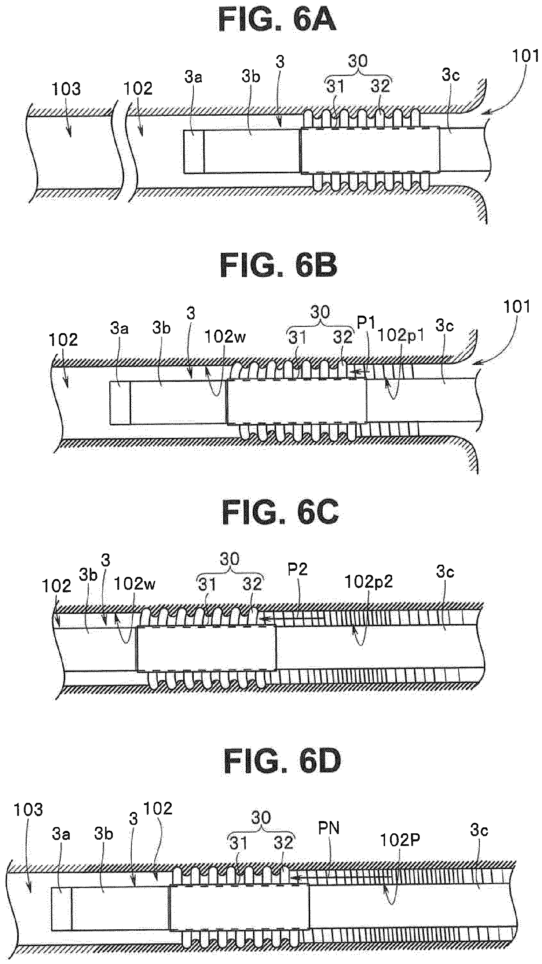

Action of the attachment unit 30 is explained with reference to Figs. 6A to 6D.

In the present embodiment, the surgeon performs hand-side operation and inserts the insertion section 3, for example, from an anus 101 shown in Fig. 6A toward a depth 103 of a large intestine 102, a depth of a not-shown small intestine, or the like while observing an endoscopic image displayed on the monitor 13.

At this point, the surgeon operates a changeover switch of the foot switch section 43 according to necessity to advance the insertion section 3 while obtaining the first propulsion.

The attachment unit 30 shown in Fig. 6B advances while giving the first propulsion to the insertion section 3 and drawing in a lumen wall 102w of the large intestine 102. At this point, the drawn-in lumen wall 102w is compressed while being collected to the proximal end side of the attachment unit 30 to be a lumen-wall compressed section 102p1. Reaction P1 from the lumen-wall compressed section 102p1 returning to an original state acts toward the attachment unit 30 from the lumen-wall compressed section 102p1.

The insertion section 3 further advances with the first propulsion, whereby the lumen wall 102w is further drawn in to the proximal end side than the attachment unit 30 as shown in Fig. 6C. A compressed lumen-wall compressed section 102p2 is provided. Reaction P2, which is a force amount larger than the reaction P1 from the lumen-wall compressed section 102p1 returning to the original state, acts toward the attachment unit 30 from the lumen-wall compressed section 102p2.

As the insertion section 3 is advanced by the first propulsion, a lumen-wall compressed section drawn in to the proximal end side of the attachment unit 30 increases and reaction from the lumen-wall compressed section returning to an original state acting toward the attachment unit 30 from the lumen-wall compressed section also increases.

As shown in Fig. 6D, the distal end portion 3a of the insertion section 3 reaches the depth 103, which is a target part. At this point, a lumen-wall compressed section 102P is provided on the proximal end side of the attachment unit 30. Reaction PN acts toward the attachment unit 30 from the lumen-wall compressed section 102P.

In the present embodiment, the fin section 32 of the attachment unit 30 is set to fall to the distal end side with the first force amount. The first force amount is set to a force amount larger than reaction PN assumed in advance.

As a result, in a state in which the distal end portion 3a of the insertion section 3 reaches the depth 103, the fin section 32 of the attachment unit 30 is prevented from being brought down by the reaction PN acting toward the attachment unit 30 from the lumen-wall compressed section 102P.

Therefore, the fin section 32 can continue to hold, in a compressed state, the lumen-wall compressed section 102P located on the proximal end side of the fin section 32. In other words, it is possible to prevent a situation in which, before the distal end portion 3a of the insertion section 3 reaches the depth 103, the fin section 32 of the attachment unit 30 is brought down by reaction acting toward the attachment unit 30 from the lumen-wall compressed section 102P, the lumen-wall compressed section compressed and disposed on the proximal end side of the attachment unit 30 climbs over the fin section 32, and the insertion section 3 is returned to an original position.

Note that the fin section 32 is set to fall to the proximal end side with the second force amount. The second force amount is set to a force amount acting on the fin section 32 from the lumen wall when the fin section 32 passes, with the first propulsion, a small-diameter lumen having a dimension decided in advance.

In this way, the fin member 60 is bonded and fixed to the tube main body 31 with the first adhesive 71 and the second adhesive 72, whereby the hard fixed section 70H is provided on the distal end side of the fin section 32 and the soft fixed section 70S is provided on the proximal end side.

The first force amount for bringing down the fin section 32 to the distal end side is set to a force amount larger than the reaction PN assumed in advance. For this reason, it is possible to prevent the fin section 32 from being brought down to the distal end side by the reaction PN from the lumen-wall compressed section 102P disposed in the compressed state on the proximal end side of the fin section 32 and advance the distal end portion 3a of the insertion section 3 toward a target depth while giving the first propulsion to the insertion section 3 from the attachment unit 30.

When the insertion section 3 is advanced by the first propulsion and when a force amount larger than the second force amount is caused to act on the proximal end side of the fin section 32 from a body cavity wall, the fin section 32 is bent to the proximal end side and the insertion section 3 can smoothly advance.

Note that the first force amount is set to a value set as appropriate according to a distance to a target part into which the insertion section is inserted, a state of a tube section into which the insertion section is inserted, and the like assuming reaction of the compressed lumen wall returning to an original state and with reference to the reaction.

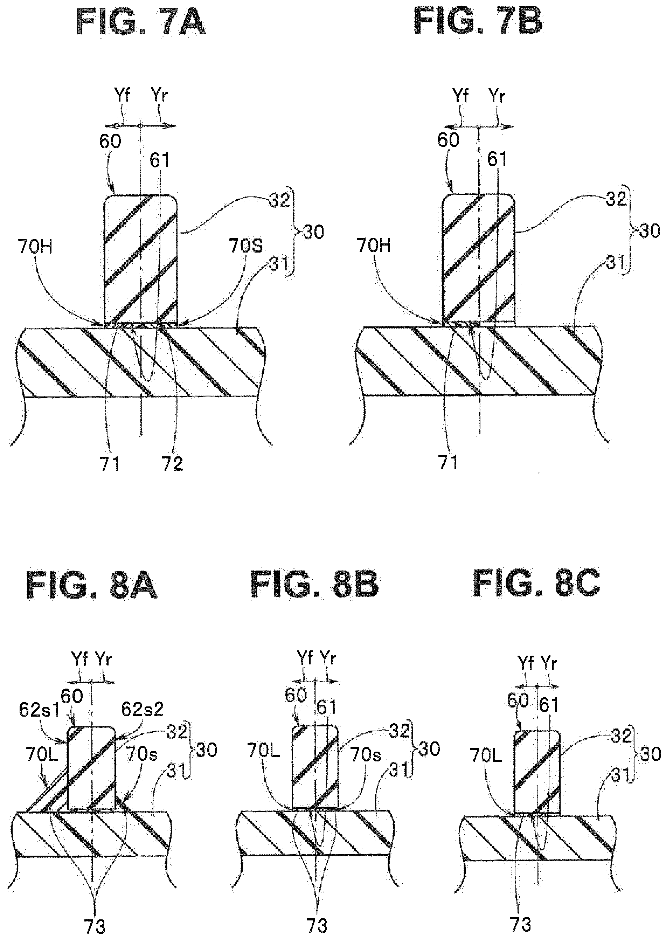

In the embodiment explained above, the fin section 32 is configured by applying the first adhesive 71 to the first side surface 62s1 side of the fin member 60 and applying the second adhesive 72 to the second side surface 62s2 side to bond and fix the fin member 60 to the tube main body 31. However, the fin section 32 that less easily falls to the distal end side with the reaction from the lumen-wall compressed section in the compressed state and easily falls to the proximal end side taking into account insertability of the insertion section may be configured as shown in Fig. 7A to Fig. 10D referred to below.

The fin section 32 shown in Fig. 7A is configured by applying the first adhesive 71 along the longitudinal axis on a distal end side of the fixed surface 61 and applying the second adhesive 72 along the longitudinal axis on a proximal end side Yr of the fixed surface 61 to bond and fix the fin member 60 to the outer surface of the tube main body 31.

With this configuration, the distal end side of the fin section 32 is the hard fixed section 70H and the proximal end side of the fin section 32 is the soft fixed section 70S. As a result, the fin section 32 less easily falls to the distal end side and easily falls to the proximal end side. Consequently, it is possible to obtain the same action and effects as the action and effects in the embodiment explained above.

Note that, as shown in Fig. 7B, the fin section 32 may be configured by applying the first adhesive 71 along the longitudinal axis only on the distal end side of the fixed surface 61 to bond and fix the fin member 60 to the outer surface of the tube main body 31. As a result, the fin section 32 less easily falls to the distal end side and easily falls to the proximal end side. Therefore, it is possible to obtain the same action and effects as the action and effects in the embodiment explained above.

The fin member 60 configuring the fin section 32 shown in Figs. 8A to 8C is fixed to the outer surface of the tube main body 31; as shown in Fig. 8A, an adhesive 73 is applied along the longitudinal axis on the first side surface 62s1 side and the second side surface 62s2 side of the fin member 60. The fin member 60 is bonded and fixed to the outer surface of the tube main body 31 by the adhesive 73 to be configured as the fin section 32.

In the present embodiment, a sectional area of an adhesive hardened section provided on the distal end side of the fin section 32 and a sectional area of an adhesive hardened section provided on the proximal end side are different. More specifically, a sectional area of a first fixed section, which is the adhesive hardened section on the distal end side, is larger than a sectional area of a second fixed section, which is the adhesive hardened section on the proximal end side, in advance.

In this way, a large fixed section 70L functioning as the first fixed section having the large sectional area is provided on the distal end side of the fin section 32. A small fixed section 70s functioning as a second fixed section having a sectional area smaller than the sectional area of the large fixed section 70L is provided on the proximal end side.

As a result, the fin section 32 including the large fixed section 70L and the small fixed section 70s less easily falls to the distal end side and easily falls to the proximal end side. Therefore, it is possible to obtain the same action and effects as the action and effects in the embodiment explained above.

It is possible to perform the adjustment of the less easiness of the falling to the distal end side and the easiness of the falling to the proximal end side of the fin section 32 to cope with an assumed force amount by setting a size of the sectional area of the large fixed section 70L and a size of the sectional area of the small fixed section 70s as appropriate.

Note that the adhesive 73 may be applied to the fixed surface 61 to bond and fix the fin member 60 to the tube main body 31. In this case, as shown in Fig. 8B, the large fixed section 70L, in which the area of the adhesive hardened section is large, is provided on the distal end side Yf of the fixed surface 61 and the small fixed section 70s, in which the area of the adhesive hardened section is smaller than the large fixed section 70L, is provided on the proximal end side Yr.

With this configuration, the fin section 32 less easily falls to the distal end side and easily falls to the proximal end side. It is possible to obtain the same action and effects as the action and effects in the embodiment.

As shown in Fig. 8C, the adhesive 73 may be applied only to the distal end side of the fixed surface 61 to bond and fix the fin member 60 to the outer surface of the tube main body 31. As a result, the fin section 32 less easily falls to the distal end side and easily falls to the proximal end side. It is possible to obtain the same action and effects as the action and effects in the embodiment explained above.

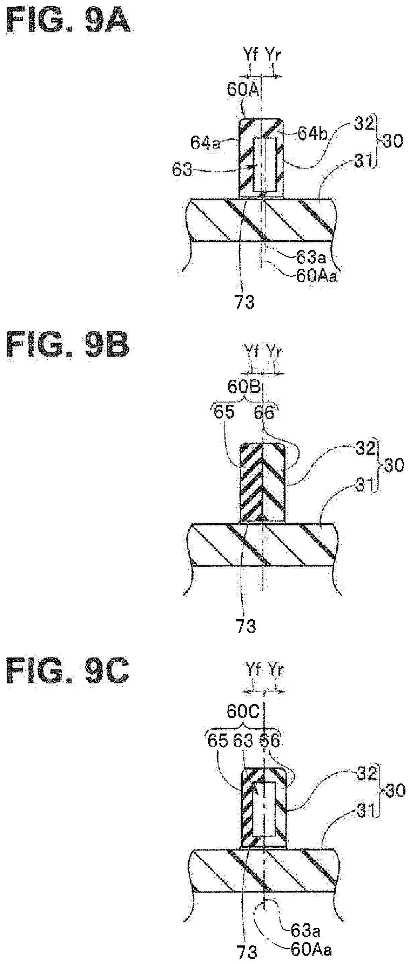

In Fig. 9A to Fig. 9C, the fin section 32 is configured by bonding and fixing fin members 60A, 60B, and 60C different from the fin member 60 to the tube main body 31. In these figures, the fin members 60A, 60B, and 60C are bonded and fixed to the outer surface of the tube main body 31 by one kind of, for example, the adhesive 73 applied to the fixed surface 61.

The fin member 60A shown in Fig. 9A is an elongated elastic member made of, for example, rubber having flexibility and elasticity decided in advance and includes a hollow section 63 having a sectional shape decided in advance along an extending direction of a spiral direction. In the figure, the hollow section 63 is a through-hole along the extending direction of the spiral direction. However, the hollow section 63 may be a longitudinal direction groove along the extending direction. An opening of the groove is provided on the fixed surface 61 side.

Since a thick section 64a and a thin section 64b are provided in the fin member 60A, a hollow-section center line 63a of the hollow section 63 and a fin-member center line 60Aa positionally deviate.

In the present embodiment, the hollow-section center line 63a positionally deviates further to the proximal end side than a fin-section center line 32a such that the thick section 64a is provided on the distal end side of the fin section 32 and the thin section 64b is provided on the proximal end side.

In this way, the fin member 60A provided with the hollow section 63 is bonded and fixed to the tube main body 31 to configure the fin section 32. Consequently, the fin section 32 less easily falls to the distal end side where the thick section 64a is provided and easily falls to the proximal end side where the thin section 64b is provided. It is possible to obtain the same action and effects as the action and effects in the embodiment explained above.

Note that, in the fin member 60A explained above, it is possible to perform the adjustment of the less easiness of the falling to the distal end side and the easiness of the falling to the proximal end side of the fin section 32 to cope with an assumed force amount by setting an opening width of the hollow section 63 as appropriate or setting a positional deviation amount between the hollow-section center line 63a and the fin-member center line 60Aa as appropriate.

More specifically, when the opening width of the hollow section 63 is formed small and the hollow-section center line 63a is caused to further positionally deviate in the proximal end side, thickness of the thick section 64a further increases and, on the other hand, thickness of the thin section 64b decreases. It is possible to make the fin section 32 less easily fall to the distal end side.

The fin member 60B shown in Fig. 9B is configured as an elongated elastic member having flexibility and elasticity decided in advance by integrating a first elastic member 65 and a second elastic member 66, which are two elastic members.

The first elastic member 65 is a hard tabular elastic member with high rigidity having flexibility and elasticity decided in advance. The second elastic member 66 is a soft tabular elastic member with low rigidity compared with the first elastic member 65. In the present embodiment, the first elastic member 65 and the second elastic member 66 have the same shape.

In this way, the fin member 60B configured by integrating the first elastic member 65 and the second elastic member 66 is bonded and fixed to the tube main body 31 to configure the fin section 32. With this configuration, the fin section 32 less easily falls to the distal end side where the first elastic member 65 is provided and easily falls to the proximal end side where the second elastic member 66 is provided. It is possible to obtain the same action and effects as the action and effects in the embodiment explained above.

Note that it is possible to form the first elastic member 65 and the second elastic member 66 in different shapes to adjust the less easiness of the falling to the distal end side and the easiness of the falling to the proximal end side as appropriate. More specifically, it is possible to make the fin section 32 less easily fall to the distal end side by increasing the thickness of the first elastic member 65 and reducing the thickness of the second elastic member 66 without changing the thickness of the fin member 60.

In the fin member 60B explained above, it is possible to perform the adjustment of the less easiness of the falling to the distal end side and the easiness of the falling to the proximal end side as appropriate by adjusting a characteristic of the first elastic member 65 and a characteristic of the second elastic member 66 as appropriate.

Further, as shown in Fig. 9C, the hollow section 63 may be provided in the fin member 60B to configure a fin member 60C. The hollow-section center line 63a of the hollow section 63 coincides with the fin-member center line 60Aa.

With this configuration, it is possible to perform the adjustment of the easiness of the less falling to the distal end side and the easiness of the falling to the proximal end side of the fin section 32 configured of the fin member 60C to cope with an assumed force amount by changing the opening width of the hollow section 63 provided in the fin member 60B in addition to a combination of the first elastic member 65 and the second elastic member 66 configuring the fin member 60B.

Note that, in the embodiment shown in Fig. 8A to Fig. 9C, the fin member is bonded and fixed to the tube main body. However, the fin member may be integrally fixed to the tube main body by welding.

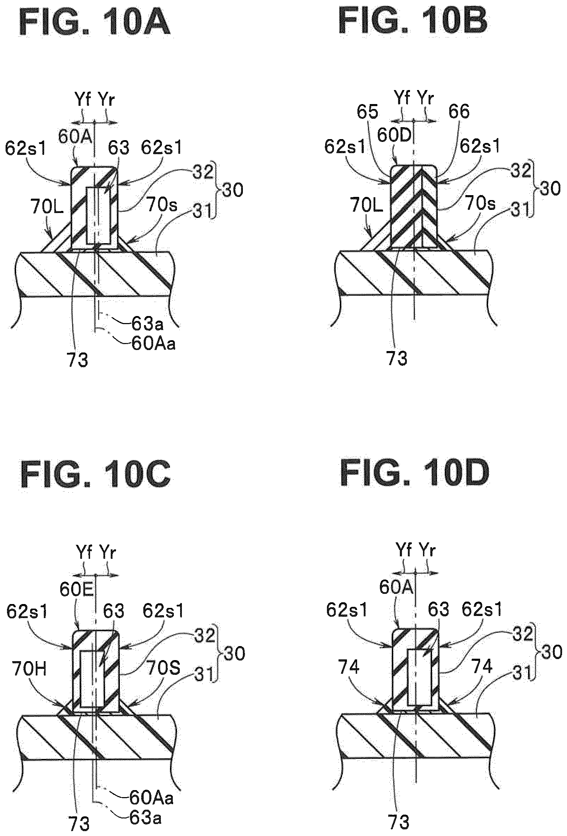

The adjustment of the less easiness of the falling to the distal end side and the easiness of the falling to the proximal end side of the fin section 32 may be performed by combining the techniques explained above as shown in Fig. 10A to Fig. 10D.

In Fig. 10A, the fin member 60A is bonded and fixed to the outer surface of the tube main body 31 by the large fixed section 70L of the adhesive 73 applied to the first side surface 62s1 and the small fixed section 70s of the adhesive 73 applied to the second side surface 62s2.

With this configuration, it is possible to perform the adjustment of the less easiness of the falling to the distal end side and the easiness of the falling to the proximal end side of the fin section 32 by setting, as appropriate, the opening width of the hollow section 63 of the fin member 60A, a positional deviation amount between the hollow-section center line 63a of the hollow section 63 and the fin-member center line 60Aa, the size of the sectional area of the large fixed section 70L, and the size of the sectional area of the small fixed section 70s.

A fin member 60D shown in Fig. 10B is a modification of the fin member 60B. As explained above, the first elastic member 65 and the second elastic member 66 have different shapes. The thickness of the first elastic member 65 is formed thick and the thickness of the second elastic member 66 is formed thin.

As in Fig. 10A, the fin member 60D is bonded and fixed to the outer surface of the tube main body 31 by the large fixed section 70L and the small fixed section 70s.

With this configuration, it is possible to perform the adjustment of the less easiness of the falling to the distal end side and the easiness of the falling to the proximal end side of the fin section 32 by setting the size of the sectional area of the large fixed section 70L and the size of the sectional area of the small fixed section 70s as appropriate in addition to the thickness of the first elastic member 65 of the fin member 60D and the thickness of the second elastic member 66.

A fin member 60E shown in Fig. 10C includes the hollow section 63. The hollow-section center line 63a of the hollow section 63 positionally deviates further to the proximal end side than the fin-section center line 32a oppositely to the above description.

The fin member 60E is bonded and fixed to the outer surface of the tube main body 31 by the hard fixed section 70H provided on the distal end side and the soft fixed section 70S provided on the proximal end side.

With this configuration, it is possible to perform the adjustment of the less easiness of the falling to the distal end side and the easiness of the falling to the proximal end side of the fin section 32 by setting the opening width of the hollow section 63 of the fin member 60E and the positional deviation amount between the hollow-section center line 63a of the hollow section 63 and the fin-member center line 60Aa as appropriate and selecting a type of the first adhesive 71 configuring the hard fixed section 70H and a type of the second adhesive 72 configuring the soft fixed section 70S as appropriate.

As shown in Fig. 10D, bonding and fixing sections 74 are provided on the first side surface 62s1 side and the second side surface 62s2 side of the fin member 60A shown in Fig. 9A to bond and fix the fin member 60A to the outer surface of the tube main body 31.

With this configuration, it is possible to perform the adjustment of the less easiness of the falling to the distal end side and the easiness of the falling to the proximal end side of the fin section 32 by setting the opening width of the hollow section 63 of the fin member 60A, the positional deviation amount between the hollow-section center line 63a of the hollow section 63 and the fin-member center line 60Aa, and a size of a sectional area of the bonding and fixing sections 74 as appropriate.

Note that, in the embodiment explained above, the fin is configured by providing the fin member in the tube main body. However, the fin and the tube main body may be integrally configured by two-color molding.

In the embodiment explained above, the fin member 60 is disposed in an erected state on the outer surface of the tube main body 31. However, the fin member 60 may be tilted in a proximal end direction of the insertion section 3 and provided in the tube main body 31 to configure the fin section 32. With this configuration, the fin section 32 less easily falls to the distal end side and easily falls to the proximal end side.

Note that the present invention is not limited only to the embodiment explained above and can be variously modified and carried out in a range not departing from the spirit of the invention. The insertion device is not limited to the endoscope and may be, for example, a treatment instrument for endoscope inserted through a treatment instrument channel of an endoscope or a guide tube that guides an endoscope into a body. In this case, the attachment unit is attached to an insertion section of the treatment instrument for endoscope or an insertion section of the guide tube.

This application is filed claiming priority from Japanese Patent Application No. 2015-113342 filed in Japan on June 3, 2015, disclosed contents of which are incorporated in this specification, the claims, and the drawings.

## Claims

1. An attachment unit comprising:
a unit main body attached to an insertion section of an insertion device and disposed to be rotatable around a longitudinal axis of the insertion section; and
an elastic convex section protrudingly provided on an outer circumferential surface of the unit main body and spirally extended along a longitudinal axis of the unit main body, wherein
in the elastic convex section, a first force amount necessary for bringing down the elastic convex section toward a distal end side of the insertion section and a second force amount necessary for bringing down the elastic convex section toward a proximal end side, which is an opposite direction of the distal end side, are different, and
in the elastic convex section provided on the unit main body, the first force amount is larger than the second force amount.

2. The attachment unit according to claim 1, wherein the elastic convex section is an elongated and solid elastic member having flexibility and elasticity decided in advance and is fixed to the outer circumferential surface of the unit main body.

3. The attachment unit according to claim 2, wherein
the elastic convex section is provided by the elastic member fixed in an erected state on the outer circumferential surface of the unit main body by a first adhesive and a second adhesive different from the first adhesive,
the elastic convex section provided on the outer circumferential surface of the unit main body includes a first hardened section formed by hardening of the first adhesive on a distal end side of the elastic convex section and includes a second hardened section formed by hardening of the second adhesive on a proximal end side of the elastic convex section, and
hardness of the first hardened section is higher than hardness of the second hardened section.

4. The attachment unit according to claim 2, wherein
the elastic convex section is provided by the elastic member fixed in an erected state on the outer circumferential surface of the unit main body by bonding or welding,
the elastic convex section provided on the outer circumferential surface of the unit main body includes a first fixed section on the distal end side of the elastic convex section and includes a second fixed section on the proximal end side of the elastic convex section, and
a sectional area of the first fixed section is larger than a sectional area of the second fixed section.

5. The attachment unit according to claim 2, wherein a hollow section along a longitudinal axis is provided in the elastic member.

6. The attachment unit according to claim 5, wherein, in the hollow section, thickness on the distal end side of the elastic convex section and thickness on the proximal end side are different.

7. The attachment unit according to claim 2, wherein the elastic member is formed by integrating two kinds of tabular elastic members.

8. The attachment unit according to claim 7, wherein
the two kinds of tabular members have a same shape, and
rigidity of a first tabular elastic member provided on the distal end side of the elastic convex section is higher than rigidity of a second tabular elastic member provided on the proximal end side of the elastic convex section.

9. The attachment unit according to claim 7, wherein
the two kinds of tabular members have different thicknesses of the tabular members, and
thickness of the first tabular elastic member provided on the distal end side of the elastic convex section is larger than thickness of the second tabular elastic member provided on the proximal end side of the elastic convex section.

10. The attachment unit according to claim 1, wherein the elastic convex section inclines to the proximal end side to be provided on the outer circumferential surface of the unit main body.

11. The attachment unit according to claim 1, wherein the first force amount is larger than a force amount assumed in advance of a lumen wall compressed and disposed on the proximal end side of the elastic convex section returning to an original state.
